# EUROPEAN PATENT APPLICATION

(11) **EP 4 635 512 A1**
(43) Date of publication of application: **22.10.2025**
(21) Application number: 25153521.7
(22) Date of filing: 23.01.2025
(51) Int. Cl.: A61K 40/11, A61K 40/31, A61K 40/41, A61K 40/42

(54) **APPLICATION OF CAR-T CELL IN SUPPRESSING CENTRAL NERVOUS SYSTEM INFLAMMATION**

(30) Priority: 15.04.2024 CN 202410448166
(71) Applicant: Tongji Hospital Affiliated with Tongji Medical College of Huazhong University of Science And Technology, Wuhan City Hubei Province 430030 (CN)
(72) Inventor: QIN, Chuan, Wuhan City, 430030 (CN); TIAN, Daishi, Wuhan City, 430030 (CN); WANG, Wei, Wuhan City, 430030 (CN); DONG, Minghao, Wuhan City, 430030 (CN); ZHOU, Luoqi, Wuhan City, 430030 (CN); PANG, Xiaowei, Wuhan City, 430030 (CN); XIAO, Jun, Wuhan City, 430030 (CN); SHANG, Ke, Wuhan City, 430030 (CN); YANG, Sheng, Wuhan City, 430030 (CN); ZHANG, Hang, Wuhan City, 430030 (CN); CHU, Yunhui, Wuhan City, 430030 (CN)
(74) Representative: Chung, Hoi Kan

(57) **Abstract**

The present invention discloses an application of a CAR-T cell in suppressing central nervous system inflammation in the technical field of monitoring, diagnosis, and treatment of central nervous system diseases. The CAR-T cell targets B-cell maturation antigens to: suppress expression of chemokines CCL1, CCL3, CCL19, CCL22, CCL24, CXCL1, CXCL5, CXCL8, and CXCL13 in cerebrospinal fluid; and/or suppress expression of inflammatory factors IL-2, IL-6, TNF-α, and IFN-γ; and/or suppress expression of complement components C3 and C5a; and/or suppress activation of CD4+T cells, CD8+T cells, NK cells, and myeloid cells. The present invention further provides a method for suppressing central nervous system inflammation using the aforementioned CAR-T cell, and a corresponding product for suppressing central nervous system inflammation.

## Description

### TECHNICAL FIELD

The present invention relates to the technical field of monitoring, diagnosis, and treatment of central nervous system diseases, and in particular, to an application of a chimeric antigen receptor (CAR)-T cell in suppressing central nervous system inflammation.

### BACKGROUND

The central nervous system (CNS) is a main part of the human nervous system composed of the brain and spinal cord. Over the past decades of research, it was generally believed that the blood-brain barrier provided a homeostatic environment for the brain by blocking peripheral immune cells and humoral immune factors in the central nervous system. However, in recent years, accumulating evidence (for example, Zang,X., Chen,S., Zhu,J., Ma,J. & Zhai,Y (2022). The Emerging Role of Central and Peripheral Immune Systems in Neurodegenerative Diseases. Frontiers in aging neuroscience) supports the existence of disorder of the immuno-inflammatory microenvironment in central nervous system diseases.

The immuno-inflammatory microenvironment is thought to play a key role in regulating central nervous system homeostasis and diseases. Cerebrovascular disease is the most common type of central nervous system disease, and immuno-inflammatory activation plays an extremely important role in its pathogenesis. Due to disruption of the blood-brain barrier, central nervous system antigens that are normally immunologically sequestered come into contact with the immune system, inducing an autoimmune response. An imbalanced immune microenvironment may further induce inflammatory responses and cause secondary tissue damage. In some degenerative diseases of the central nervous system, such as amyotrophic lateral sclerosis (ALS), Alzheimer's disease (AD), and Parkinson's disease (PD), inflammatory dysregulation of the central nervous system also plays an important role in pathological development.

With the advancement of neuroimmunology, researchers have gradually deepened their understanding of related diseases directly involved in immune response, especially autoimmune diseases of the central nervous system. Such diseases include neuromyelitis optica spectrum disorder (NMOSD), myelin oligodendrocyte glycoprotein antibody-associated disease (MOGAD), multiple sclerosis (MS), acute disseminated encephalomyelitis (ADEM), autoimmune encephalitis (AE), central nervous system vasculitis, etc. Their pathogenesis is complex and mainly caused by autoimmune cells, autoantibodies, and other immune molecules directly or indirectly attacking the nervous system (including neurons, glial cells, and myelin sheaths), thereby further causing immuno-inflammatory responses.

Neuromyelitis optica spectrum disorder (NMOSD) is an autoimmune inflammatory demyelinating disease of the central nervous system (CNS) that can affect the spinal cord, optic nerve, area postrema, brainstem, diencephalon, and brain, with optic neuritis and long-segment transverse myelitis more common. The pathogenesis of NMOSD is mainly as follows: the main pathogenic antibody of NMOSD, namely aquaporin 4 (AQP4)-IgG, induces astrocytes expressing AQP4 to produce a pro-inflammatory factor interleukin-6 (IL-6); the IL-6 promotes the differentiation of Th17 cells and blocks the activation of regulatory T cells; and the IL-6 also stimulates the differentiation of plasma cells to produce the AQP4-IgG, destroying the integrity of the blood-brain barrier, thereby promoting the AQP4-IgG, pro-inflammatory factor, and inflammatory cells to enter the central nervous system. Once the AQP4-IgG entering the central nervous system binds to the AQP4 antigen on the astrocytes, complement-mediated astrocyte damage occurs first, followed by granulocyte infiltration, oligodendrocyte death, demyelination, and ultimately neuronal cell death. In addition, some studies have shown that immune cells in NMOSD can participate in the pathogenesis of NMOSD by regulating pro-inflammatory and anti-inflammatory cytokines. The regulatory dysfunction of immune cells may lead to the activation of a large number of pro-inflammatory factors, immune imbalance, and ultimately tissue damage. Therefore, the regulation of the inflammatory environment of the central nervous system is crucial for the progression of NMOSD.

Currently, the treatment of NMOSD includes acute phase treatment such as glucocorticoid pulse therapy, plasma exchange, and immunoglobulin therapy, and preventive treatment mainly using immunosuppressive agents and monoclonal antibodies. Despite substantial progress in the treatment of NMOSD, a subset of patients with refractory and relapsed disease still respond poorly to existing therapies, possibly because existing therapies do not effectively target the inherent immune dysregulation of the central nervous system.

Chimeric antigen receptor T-cell (CAR-T) immunotherapy is a type of immunotherapy mainly used for the clinical treatment of patients with hematological malignancies and malignant tumors. CAR-T cell immunotherapy is implemented by collecting and isolating T cells from a patient's blood, and then genetically modifying them and culturing and amplifying them in large quantities in vitro, and finally infusing them back into the patient's body to eliminate target cells in a targeted manner. Due to its capability of directly inducing target cell death and self-amplification, CD19-targeted CAR-T cell immunotherapy has been confirmed by researchers to treat primary central nervous system lymphoma and show efficacy and safety in patients with relapsed acute lymphoblastic leukemia and central nervous system involvement. In addition, BCMA-targeted CAR-T cell immunotherapy can effectively alleviate the degree of disability and disease recurrence in patients with relapsed/refractory AQP4 IgG seropositive NMOSD. Although CAR-T cells have shown beneficial effects in some tumors involving the central nervous system and autoimmune diseases of the central nervous system, there is still a lack of application of CAR-T cell immunotherapy in regulating neuroinflammatory responses in central nervous system diseases. Since inflammatory activation plays a very important role in the pathogenesis of central nervous system diseases, the application of CAR-T cell immunotherapy in the treatment of central nervous system diseases by suppressing central nervous system inflammation is crucial.

### SUMMARY

The present invention provides a method for suppressing central nervous system inflammation, a CAR-T cell used to suppress central nervous system inflammation, and related products and application methods. The objectives of the present invention are achieved by the following technical solutions.

A method for suppressing central nervous system inflammation using a CAR-T cell that is not aimed at disease diagnosis and treatment, where the CAR-T cell targets B-cell maturation antigens to: suppress expression of chemokines CCL1, CCL3, CCL19, CCL22, CCL24, CXCL1, CXCL5, CXCL8, and CXCL13 in cerebrospinal fluid;
and/or suppress expression of inflammatory factors IL-2, IL-6, TNF-α, and IFN-γ;
and/or suppress expression of complement components C3 and C5a;
and/or suppress activation of CD4+T cells, CD8+T cells, NK cells, and myeloid cells.

Furthermore, the CAR-T cell in the aforementioned method is derived from peripheral blood mononuclear cells (PBMCs) isolated from peripheral blood.

The present invention further provides a product for suppressing central nervous system inflammation based on the aforementioned CAR-T cell.

Accordingly, the present invention further provides a product for detecting central nervous system inflammation, where the product is used to: detect expression of chemokines CCL1, CCL3, CCL19, CCL22, CCL24, CXCL1, CXCL5, CXCL8, and CXCL13 in cerebrospinal fluid;
and/or detect expression of inflammatory factors IL-2, IL-6, TNF-α, and IFN-γ in cerebrospinal fluid;
and/or detect expression of complement components C3 and C5a in cerebrospinal fluid;
and/or detect activation of CD4+T cells, CD8+T cells, NK cells, and myeloid cells in cerebrospinal fluid.

Furthermore, the product is a test kit, test strip, or detection chip.

The present invention further provides a marker for detecting and evaluating the immune microenvironment of the central nervous system, where the marker contains chemokines CCL1, CCL3, CCL19, CCL22, CCL24, CXCL1, CXCL5, CXCL8, and CXCL13;
and/or the marker contains inflammatory factors IL-2, IL-6, TNF-α, and IFN-γ;
and/or the marker contains complement components C3 and C5a.

The applicant of the present invention detected the levels of the inflammatory factors, chemokines, and complement components in the cerebrospinal fluid of the control group and NMOSD patients at baseline and 3 months after infusion of the CAR-T product and found that the level of inflammation in myeloid cells in the cerebrospinal fluid showed significant activation. Specifically, it was found that: (1) the expression of the pro-inflammatory cytokines interleukin 2 (IL-2), interleukin 6 (IL-6), interferon gamma (IFN-γ), and tumor necrosis factor (TNF-α) after CAR-T cell immunotherapy was significantly reduced; (2) the expression of the chemokines CCL1 (P=0.006), CCL3 (P=0.0006), CCL19 (P=0.01), CCL22 (P=0.04), CCL24 (P=0.002), CXCL1 (P=0.04), CXCL5 (P=0.03), CXCL8 (P=0.007), and CXCL13 (P=0.03) after CAR-T cell immunotherapy was significantly reduced; and (3) the levels of the complement components C5a and C3 after CAR-T cell immunotherapy were significantly reduced.

Therefore, the level of inflammatory activation in the central nervous system can be determined based on the levels of expression of the chemokines CCL1, CCL3, CCL19, CCL22, CCL24, CXCL1, CXCL5, CXCL8, and CXCL13, or based on the levels of expression of the inflammatory factors IL-2, IL-6, TNF-α, and IFN-γ, or based on the levels of expression of the complement components C3 and C5a.

When conducting the aforementioned CAR-T cell immunotherapy on representative NMOSD patients, it was found that the immunotherapy could improve the inflammatory environment of the central nervous system by regulating the strength of interaction between immune cells in the cerebrospinal fluid to suppress the macrophage-like cell inflammation, chemotaxis, and complement pathways in the cerebrospinal fluid, and by regulating the inflammation, chemotaxis, and complement pathway of cerebrospinal fluid to suppress pro-inflammatory cytokines, chemokines, and/or complement components. Therefore, the CAR-T cell immunotherapy can be used to suppress and reduce inflammation in the central nervous system.

Compared with the prior art, the present invention has the following beneficial effects: it provides a CAR-T cell and a method for using the CAR-T cell to improve and reshape the immune microenvironment of the central nervous system. By analyzing relevant indicators of cerebrospinal fluid samples after infusion of the CAR-T cell, it was found that the CAR-T cell could suppress neuroinflammation in central nervous system diseases.

### BRIEF DESCRIPTION OF THE DRAWINGS

FIG. 1 shows a difference in inflammation scores among immune cell subsets;
FIG. 2 shows a difference in the number of interactions among cerebrospinal fluid subsets before and after CAR-T cell immunotherapy;
FIG. 3 shows proportions of different myeloid cells in peripheral blood and cerebrospinal fluid;
FIG. 4 shows changes in inflammation, chemotaxis, and complement-related pathways after CAR-T cell immunotherapy;
FIG. 5 shows a difference in levels of expression of inflammatory factors after CAR-T cell immunotherapy;
FIG. 6 shows a difference in levels of expression of CCL-family chemokines after CAR-T cell immunotherapy;
FIG. 7 shows a difference in levels of expression of CXCL-family chemokines after CAR-T cell immunotherapy;
FIG. 8 shows a difference in levels of expression of complement components after CAR-T cell immunotherapy; and
FIG. 9 shows changes in CD4+T cell, CD8+T cell, NK cell, and myeloid cell-related pathways at baseline and after CAR-T cell immunotherapy.

### DETAILED DESCRIPTION OF THE EMBODIMENTS

The technical solutions of the present invention will be clearly and completely described below with reference to the accompanying drawings. Obviously, the described embodiments are only some but not all of the embodiments of the present invention. Based on the embodiments in the present invention, all other embodiments obtained by those of ordinary skill in the art without making creative efforts shall fall within the scope of protection of the present invention.

In the following embodiments, neuromyelitis optica spectrum disorder (NMOSD) was selected as a representative of inflammatory disorders of the central nervous system (CNS) for a clinical trial. The subjects of the clinical trial included: (1) 5 patients with refractory or relapsed aquaporin-4 serum antibody-positive NMOSD treated with anti-BCMA CAR-T cells, from whom cerebrospinal fluid samples were obtained separately at baseline and 3 months after infusion of the CAR-T product; and (2) 5 control subjects matched for age and gender with the NMOSD subjects, including 1 patient with primary headache, 3 patients with non-inflammatory peripheral neuropathy, and 1 patient with idiopathic intracranial hypertension, from whom blood and cerebrospinal fluid samples were obtained separately. As approved by the Institutional Review Board of Tongji Hospital, the patients provided documented informed consent for the use of their infused products, blood, and cerebrospinal fluid samples in the trial related to the following embodiments.

The CAR-T cells provided and infused in the following embodiments were CAR-T cells targeting B-cell maturation antigens (BCMAs). These CAR-T cell products used lentivirus as a gene vector to transfect autologous T cells, and the CAR contains fully human scFv, CD8a hinge and transmembrane, 4-1BB costimulation, and CD3ζ activation domains. The CAR-T cell, with a research and development code of CT103A, was prepared by Nanjing IASO Biotherapeutics Co., Ltd.

Specifically, the method for obtaining the CAR-T cells provided in the following embodiments was as follows:
(1) first, the patients' leukocytes were separated using CD3 microbeads (Miltenyi Biotec) according to the manufacturer's protocol;
(2) the isolated leukocytes were then activated using Dynabeads^{®} Human T-Activator CD3/CD28 (Invitrogen) in the CTS^{™} OpTmizer^{™} culture medium (Gibco), together with 20 mM L-GlutaMAX^{™} (Gibco) and 200 IU/mL recombinant human IL-2 (SL PHARM);
(3) the CAR-T cells obtained in the step (2) used lentivirus as a gene vector to transfect autologous T cells, and the CAR contained fully human scFv, CD8a hinge and transmembrane, 4-1BB costimulation, and CD3ζ activation domains.
The transfected cells were washed and debeaded on D5 and then cultured in G-Rex (Wilson Wolf). On D10-D11, the CAR-T cells were collected, washed, and suspended in a cryopreservation solution; and
(4) finally, the prepared CAR-T cells were aliquoted into freezing bags (Miltenyi Biotec) and frozen to -90 °C using ThermoFisher's 7451TF CRF#4. The final products were stored in a gas-phase liquid nitrogen tank (BIOBANK-22K) below -130 °C.

### Test Example 1: Relationship between the level of inflammation of myeloid cells in cerebrospinal fluid and the degree of CNS inflammation activation

### 1. Sample collection, preparation, and single-cell RNA sequencing

(1) Sterile centrifuge tubes were used to collect cerebrospinal fluid samples from the NMOSD patients at baseline and 1 month and 3 months after infusion of BCMA-targeted CAR-T cells, and also cerebrospinal fluid samples from the control subjects.
(2) The collected cerebrospinal fluid samples were centrifuged at 300×g for 10 minutes at 4 °C to obtain precipitated cells.
(3) The precipitated cells were resuspended in 60 µL of sterile phosphate buffer, and the single cell suspension was loaded onto the 10X Chromium platform for loading.
Cell Ranger was used to demultiplex and align raw RNA data and antibody-derived tag sequences for read alignment, data filtering, barcode and UMI counting, and identification of putative cells.
The feature-barcode matrix generated from this pipeline was analyzed in R using the Seurat v4.2.0 software package for subsequent single-cell data analysis.

### 2. Reflection of CNS immune microenvironment disorder by inflammatory

### activation mediated by myeloid cells in cerebrospinal fluid

In order to clarify the dominant factors of immune microenvironment disorder in autoimmune diseases of the central nervous system, we analyzed the single-cell transcriptomic data of cerebrospinal fluid cells of 5 NMOSD patients at baseline and 3 months after infusion of the CAR-T product.

By clustering cerebrospinal fluid cell subsets, we screened out immune cell subsets with different features. We further performed single-sample gene set enrichment analysis (ssGSEA) on different immune cell subsets and defined an inflammation score for each cell type based on gene expression of classical inflammatory responses to evaluate its degree of inflammatory activation.

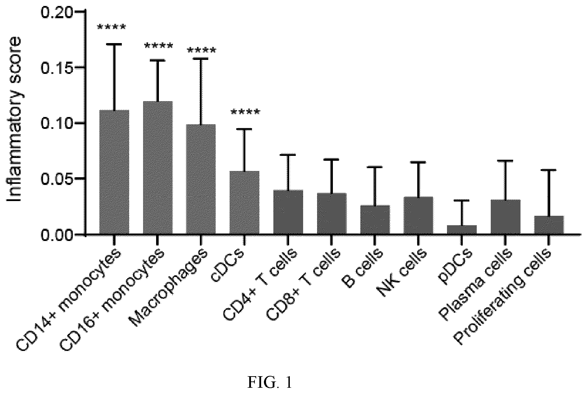

The results are shown in FIG. 1. Compared with other immune cell subsets, the inflammation scores of four subsets of myeloid cells, namely CD14+ monocytes, CD16+ monocytes (CD16+Mono), classical dendritic cells (cDCs), and macrophages, significantly increased, indicating that during the pathogenesis of NMOSD, the immune microenvironment disorder caused by inflammatory activation is mainly attributed to the inflammatory activation of myeloid cells.

Therefore, the degree of inflammatory activation in the central nervous system can be evaluated through the score of myeloid cell-mediated inflammation in the cerebrospinal fluid, providing a potential intervention target for the treatment of central nervous system diseases.

### Test Example 2: Study on the suppressive effect of CAR-T cell immunotherapy on the activation of CNS immune inflammation

### 1. Relationship between the strength of immune cell interaction in cerebrospinal fluid and neuroinflammation

In order to evaluate the level of neuroinflammation in the central nervous system after CAR-T cell immunotherapy, we used the bioinformatics tool CSO_map to analyze the interaction between different immune cell subsets in the cerebrospinal fluid at baseline and 3 months after infusion; and then we calculated changes in the number of interactions between different immune cell subsets before and after therapy (number of interactions after therapy - number of interactions at baseline / number of interactions at baseline), and compared the changes in the interaction relationship between the two conditions.

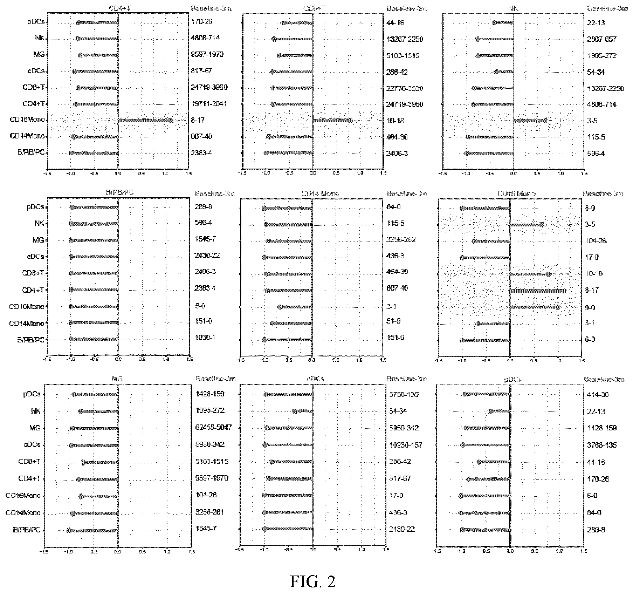

The results are shown in FIG. 2, which shows the interactions between different immune cells and other cell subsets; the blue line to the left indicates that the number of interactions between immune cells after infusion of the CAR-T product is reduced compared with that before infusion, and the yellow line to the right indicates that the number of immune cells increases after infusion of the CAR-T product; and the number on the right indicates the specific number of interactions.

As can be seen from FIG. 2, at baseline of the NMOSD patients, a strong interaction existed between various immune cell subsets in the cerebrospinal fluid, indicating that the central nervous system immune microenvironment disorder in the NMOSD patients is manifested by a strong interaction between immune cell subsets. However, the strength of interaction between most immune cell subsets in the cerebrospinal fluid showed a significant downward trend at 3 months after infusion of the CAR-T product, indicating that CAR-T cell immunotherapy can target and reshape the immune microenvironment of the central nervous system by reversing the strong interaction between immune cells in the cerebrospinal fluid, thereby achieving its effect of treating and reducing CNS inflammation.

### 2. Mechanism of CAR-T cell immunotherapy in suppressing CNS neuroinflammation

(1) Reshape the immune microenvironment of the CNS by suppressing macrophage-like cell inflammation, chemotaxis, and complement pathways in cerebrospinal fluid

Based on the fact that inflammatory activation of the central nervous system microenvironment in the NMOSD patients was mainly mediated by myeloid cells, we further used the Seruat package to classify myeloid cells in peripheral blood and cerebrospinal fluid according to their respective markers.

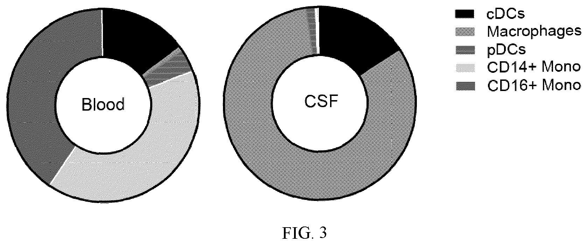

Specifically, the myeloid cells included CD16+ monocytes (CD16+ Mono), CD14+ monocytes (CD14+ Mono), classical dendritic cells (cDCs), plasmacytoid dendritic cells (pDCs), and macrophages. FIG. 3 shows proportions of different myeloid cells in peripheral blood and cerebrospinal fluid, among which macrophages account for the highest proportion of myeloid cells in the cerebrospinal fluid.

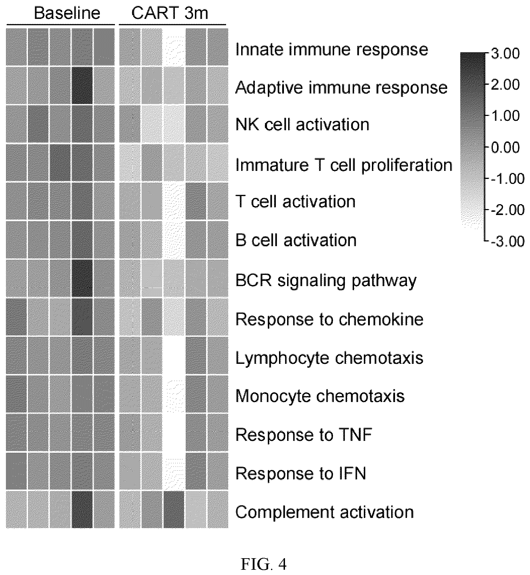

Then, we performed single-sample gene set enrichment analysis (ssGSEA) on the macrophages in the cerebrospinal fluid to determine the transcriptomic features of this type of macrophage before and after CAR-T cell immunotherapy. FIG. 4 shows changes in inflammation, chemotaxis, and complement-related pathways at baseline and after CAR-T cell immunotherapy.

Specifically, these pathways include innate immune response, adaptive immune response, NK cell activation, immature T cell proliferation, T cell activation, B cell activation, BCR signaling pathway, response to chemokine, lymphocyte chemotaxis, monocyte chemotaxis, response to tumor necrosis factor (TNF), response to interferon gamma (IPNγ), and complement activation pathways. The more red the color is, the higher the activation level, and the more blue the color is, the lower the activation level. It can be seen that the elevated features of inflammation, chemotaxis, and complement-related pathways at baseline in the NMOSD patients were significantly reduced after infusion of CAR-T cells,
indicating that for autoimmune diseases of the central nervous system, CAR-T cell immunotherapy can suppress macrophage-like cell chemotaxis, inflammation, and complement pathways in cerebrospinal fluid, thereby suppressing the level of CNS inflammation.

### (2) Suppress the level of CNS inflammation by regulating inflammatory factors, chemotaxis, and complement pathways in cerebrospinal fluid

The trial detected the cerebrospinal fluid of the control subjects and the levels of the inflammatory factors, chemokines, and complement components in the cerebrospinal fluid of the NMOSD patients at baseline and 3 months after infusion of the CAR-T product.

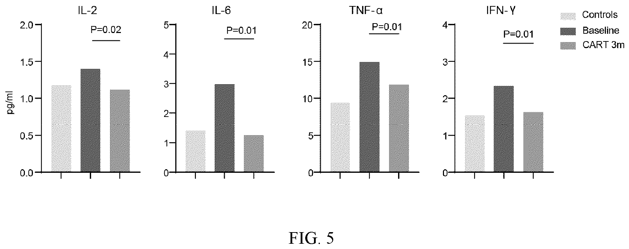

Interleukin 2 (IL-2), interleukin 6 (IL-6), interferon gamma (IFN-γ), and tumor necrosis factor (TNF-α) were detected by Luminex Assay (R&D Systems). As shown in FIG. 5, control refers to the control subjects, baseline refers to the level at baseline, and CART 3m refers to 3 months after infusion of the CAR-T product. It can be seen that the expression of pro-inflammatory cytokines IL-2 (P=0.02), IL-6 (P=0.02), TNF-α (P=0.01), and IFN-γ (P=0.01) after CAR-T cell immunotherapy was significantly reduced, indicating that CAR-T cell immunotherapy can improve the inflammatory environment of the CNS by alleviating the level of central nervous system inflammation.

Chemokines, as a large family of cytokines, are divided into four subfamilies, namely C chemokines, CC chemokines, CXC chemokines, and CX3C chemokines, based on the number and position of conserved cysteine residues at the N terminus. Specifically, the trial detected the levels of expression of CCL1, CCL3, CCL19, CCL22, CCL24, CXCL1, CXCL5, CXCL8, and CXCL13 through Luminex Assay (R&D Systems).

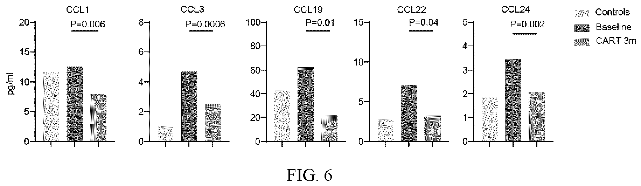

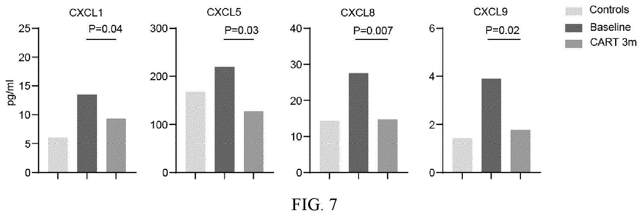

As shown in FIGs. 6 and 7, after CAR-T cell immunotherapy, the expression of the chemokines CCL1 (P=0.006), CCL3 (P=0.0006), CCL19 (P=0.01), CCL22 (P=0.04), CCL24 (P=0.002), CXCL1 (P=0.04), CXCL5 (P=0.03), CXCL8 (P=0.007), and CXCL13 (P=0.03) was significantly reduced, indicating that CAR-T cell immunotherapy can regulate the inflammatory environment of the central nervous system by suppressing the expression of chemokines in the central nervous system.

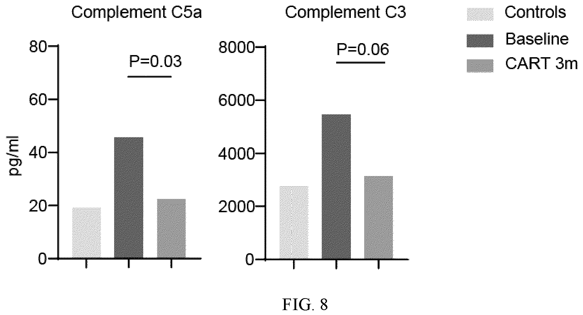

The complement system consists of more than 30 soluble proteins and membrane proteins and is an important component of the innate immune system. In central nervous system diseases, activation of the complement system is considered an important link in the neuroimmune mechanism. Various cells in the nervous system can express complement components and complement receptors, and the nervous system itself can also synthesize complement and participate in the occurrence and development of various diseases. Complement components 5a (C5a) and 3 (C3) are important components of the complement system. The trial detected the levels of expression of complement components C5a (Abcam, ab 125963) and C3 (Abcam, ab 108823) by ELISA. As shown in FIG. 8, the levels of complement components C5a and C3 decreased significantly after CAR-T cell immunotherapy, indicating that CAR-T cell immunotherapy can regulate the immune microenvironment of the central nervous system by suppressing the expression of complement components in the central nervous system and thereby affecting the complement activation pathways.

In summary, the test results of this Test Example indicate that CAR-T cell immunotherapy can alleviate neuroinflammatory damage in the central nervous system by regulating the inflammatory responses, chemotaxis pathways, and complement activation pathways of the central nervous system.

### (3) Suppress neuroinflammation by regulating pathway activation of T cells, NK cells, and myeloid cells

By performing ssGSEA analysis on the cells in the cerebrospinal fluid, we determined the transcriptomic features of CD4+T cells, CD8+T cells, NK cells, and myeloid cells before and after CAR-T cell immunotherapy.

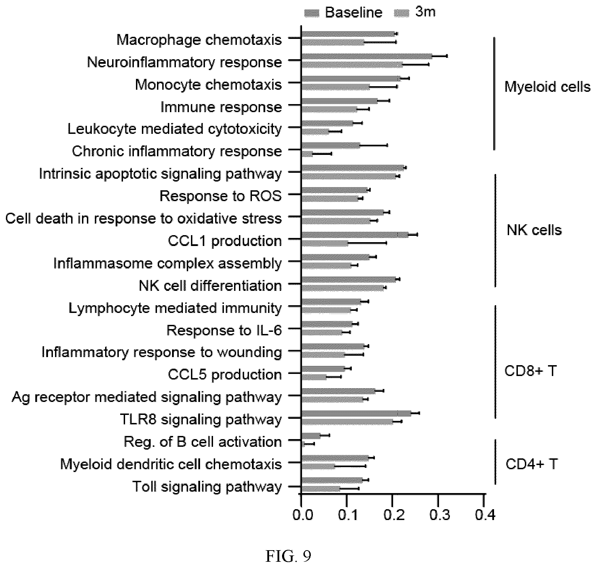

FIG. 9 shows changes in CD4+T cell, CD8+T cell, NK cell, and myeloid cell-related pathways at baseline and after CAR-T cell immunotherapy. Specifically, the Toll signaling pathway, myeloid dendritic cell chemotaxis, and regulation of B cell activation were all down-regulated in CD4+T cells after CAR-T cell immunotherapy, indicating that CAR-T cell immunotherapy alleviated the activation of CD4+T cells. The TLR8 signaling pathway, Ag receptor-mediated signaling pathway, CCL5 production, inflammatory response to wounding, response to IL-6, and lymphocyte-mediated immunity were all down-regulated in CD8+T cells after CAR-T cell immunotherapy, indicating that CAR-T cell immunotherapy alleviated the activation of CD8+T cells. The NK cell differentiation, inflammasome complex assembly, CCL1 production, cell death in response to oxidative stress, and response to reactive oxygen species (ROS) were all down-regulated in NK cells after CAR-T cell immunotherapy, indicating that CAR-T cell immunotherapy alleviated the activation of NK cells. Similarly, related pathways in myeloid cells were significantly down-regulated after CAR-T cell immunotherapy, including the intrinsic apoptosis signaling pathway, chronic inflammatory response, leukocyte-mediated cytotoxicity, immune response, monocyte chemotaxis, neuroinflammatory response, and macrophage chemotaxis, indicating that CAR-T cell immunotherapy suppressed the activation of myeloid cells.

In summary, CAR-T cell immunotherapy can alleviate the inflammatory response of the central nervous system by suppressing the activation of CD4+T cells, CD8+T cells, NK cells, and myeloid cells.

The above embodiments describe the implementation of the present invention in detail, but the present invention is not limited to the specific details in the above embodiments. Within the scope of the claims and technical concept of the present invention, various simple modifications and changes may be made to the technical solutions of the present invention, and these simple modifications and changes shall all fall within the protection scope of the present invention.

## Claims

1. A method for suppressing central nervous system inflammation using a CAR-T cell that is not aimed at disease diagnosis and treatment, wherein the CAR-T cell targets B-cell maturation antigens to: suppress expression of chemokines CCL1, CCL3, CCL19, CCL22, CCL24, CXCL1, CXCL5, CXCL8, and CXCL13 in cerebrospinal fluid;
and/or suppress expression of inflammatory factors IL-2, IL-6, TNF-α, and IFN-γ; and/or suppress expression of complement components C3 and C5a;
and/or suppress activation of CD4+T cells, CD8+T cells, NK cells, and myeloid cells.

2. The method of claim 1, wherein the CAR-T cell is derived from peripheral blood mononuclear cells (PBMCs) isolated from peripheral blood.

3. A product for suppressing central nervous system inflammation, wherein the product comprises a CAR-T cell, and the CAR-T cell targets B-cell maturation antigens to: suppress expression of chemokines CCL1, CCL3, CCL19, CCL22, CCL24, CXCL1, CXCL5, CXCL8, and CXCL13 in cerebrospinal fluid;
and/or suppress expression of inflammatory factors IL-2, IL-6, TNF-α, and IFN-γ; and/or suppress expression of complement components C3 and C5a;
and/or suppress activation of CD4+T cells, CD8+T cells, NK cells, and myeloid cells.

4. A product for detecting central nervous system inflammation, wherein the product is used to: detect expression of chemokines CCL1, CCL3, CCL19, CCL22, CCL24, CXCL1, CXCL5, CXCL8, and CXCL13 in cerebrospinal fluid;
and/or detect expression of inflammatory factors IL-2, IL-6, TNF-α, and IFN-γ in cerebrospinal fluid;
and/or detect expression of complement components C3 and C5a in cerebrospinal fluid;
and/or detect activation of CD4+T cells, CD8+T cells, NK cells, and myeloid cells in cerebrospinal fluid.

5. The product of claim 4, wherein the product is a test kit, test strip, or detection chip.

6. A marker for detecting or evaluating central nervous system inflammation, wherein the marker contains chemokines CCL1, CCL3, CCL19, CCL22, CCL24, CXCL1, CXCL5, CXCL8, and CXCL13;
and/or the marker contains inflammatory factors IL-2, IL-6, TNF-α, and IFN-γ;
and/or the marker contains complement components C3 and C5a.
